# EUROPEAN PATENT SPECIFICATION

(11) **EP 3 843 792 B1**
(45) Date of publication and mention of the grant of the patent: **28.05.2025**
(21) Application number: 19773547.5
(22) Date of filing: 29.08.2019
(51) Int. Cl.: A61L 2/04, A61K 8/02, A61L 15/44, C11D 17/04

(54) **PROCESS FOR THE STERILIZATION IN AN AUTOCLAVE OF WET PRODUCTS PACKAGED IN PLASTIC OR HEAT-SENSITIVE MATERIALS**
VERFAHREN ZUR STERILISATION IN EINEM AUTOKLAVEN VON IN KUNSTSTOFF- ODER WÄRMEEMPFINDLICHEN MATERIALIEN VERPACKTEN NASSPRODUKTEN
PROCESSUS POUR LA STÉRILISATION DANS UN AUTOCLAVE DE PRODUITS HUMIDES CONDITIONNÉS DANS DES MATÉRIAUX PLASTIQUES OU SENSIBLES À LA CHALEUR

(30) Priority: 31.08.2018 IT 201800008267
(43) Date of publication of application: 07.07.2021
(73) Proprietor: Welcare Industries S.p.A., 05018 Orvieto (TR) (IT)
(72) Inventor: DE BERNARDINI, Franco, 05020 Montecchio TR (IT); LAZZAROTTO, Fulvia, 00040 Frascati RM (IT)
(74) Representative: Di Giovine, Paolo
(86) International application number: PCT/IB2019/057269
(87) International publication number: WO 2020/044272

(56) References cited:
- WO-A1-2017/117534
- US-A- 3 348 905

## Description

### Technical field of the invention

The present invention relates to a process for sterilization by means of saturated steam, which process is effective and applicable to products that are closed in a bag, in particular to medical devices such as cloths, gauze and/or mitts soaked by an aqueous solution having detergent and/or antiseptic properties.

### Prior art

The objective of sterilization is to destroy any microorganism in the vegetative state, or spores, and is achieved by means of standardized physical and/or chemical processes, which can be repeated and can be documented. The wet steam sterilization process performed by means of an autoclave is controlled on the basis of three physical parameters - temperature, pressure, and exposure time - the control of which is fundamental for ensuring good success of the process.

The term "saturated steam" means water vapor saturated with liquid water. This condition is possible only when the liquid phase is in equilibrium with the gaseous phase. At ambient pressure this occurs only at 100°C, however it occurs theoretically at any temperature, provided the pressure is appropriate. Wet steam sterilization is performed in an autoclave. In this apparatus water vapor is used, which has the advantage of a high thermal capacity combined with the ability to penetrate into porous bodies of the devices.

In a terminal steam sterilization process, in order to obtain an SAL (sterility assurance level) of 10⁻⁶ (SAL: the probability that a single unit is not sterile of 1 in 1 million) starting from a defined level of initial contamination, according to that defined in the Official European Pharmacopoeia, the ideal relationship between these parameters is:
- temperature: 121°C;
- pressure: 1 bar;
- exposure time: 15 minutes

Right at the start of the 1990s, the European Committee of Standardization issued standards aimed at stabilizing the structural requirements on sterilization equipment for medical devices (European standard): more specifically EN 285 and EN 554 for validation of the process, the latter having since been superseded by UNI EN 17665-1 and 2.

The standardized hot steam sterilization processes have implementation temperatures between 115°C and 134°C, overpressures between 1.68 Atm and 3.05 Atm, and exposure times between 7 and 21 minutes. This sterilization method is used generally for solid bodies such as laboratory or operating theatre instruments and equipment.

At the temperatures defined by the Pharmacopoeia it is impossible to sterilize other types of products formed by heat-sensitive materials or products packaged in plastics material:
- Many components of the solutions of the medical devices containing a liquid component (for example saturated cloths or gauzes) degrade or are heavily altered.
- Notwithstanding the use of appropriately developed laminates, plastic bags experience changes and alterations of their shape.

In the prior art this type of medical device is therefore sterilized by way of other methods, such as radiation. The use of radiation, however, is not advised in a pharmaceutical environment since the substances subjected to irradiation undergo alterations anyway, to a greater or lesser degree. US 3 348 905 A and WO 2017/117534 A1 disclose methods for sterilising gauzes and impregnated wipes respectively.

The object of the present invention is to provide a process for wet sterilization under heat, which process is effective and applicable to products closed in a bag, such as some types of medical devices, for example cloths or mitts of non-woven fabric soaked in an aqueous solution having detergent or antiseptic properties.

### Glossary

In the context of the present description the term "about", when used with reference to a temperature, indicates a tolerance of ± 2°C, preferably ± 1°C, whereas when used with reference to a pressure it indicates a tolerance of ± 0.2 bar, preferably of ± 1 bar.

The term "heat-sensitive", as well as the term "thermolabile", in the context of the present description, means materials that tend to deform and/or become damaged with rising temperature. An example of thermolabile materials is constituted by the plastic materials usually used in the primary packaging of medical products or devices, such as cloths, gauze and/or mitts, for example polyethylene, which deforms beyond 90°C. Polyethylene therefore is not suitable for the primary packaging of medical devices for the skin, since these are subjected to a sterilization process at temperatures greater than 90°C.

The term "heat-resistant", in the context of the present description, means resistance to a temperature of about 105°C, without deformation or damage. For example, the material used for the preparation of the heat-sealed packaging with facilitated opening described herein can be a multi-layer plastic film with an aluminum layer or composite laminate packaging material which comprises a cast material (or obtained by means of a cast extrusion process) which, when exposed to a temperature of 105°C, does not undergo any kind of alteration of its physico-chemical properties. In other words, thanks to the content of the cast material, for example cast polypropylene, it is possible to counteract the deformation of the packaging during the sterilization process caused by temperature and pressure.

The term "mitt" in the context of the present invention means a type of medical device in the form of a glove without fingers for treatment of the skin.

### Summary of the invention

The invention described herein by means of the introduction of modifications to the known procedures makes it possible to achieve both sterility of the product and resistance thereof to the sterilization process. The modifications brought to the process make it possible to apply the saturated steam sterilization also to medical devices that, before now, could not be processed due to the deterioration of their properties and functions.

The innovation introduced by the present invention consists of the application of a saturated steam sterilization to various products among those typically subjected to this treatment. The reasons as to why the process is effective on this type of product lie in the fact that it is not the steam generated by the autoclave that realizes the process of sterilization within the product, given its penetrative power, but instead the steam produced within the bag, since this is a closed system. The steam of the autoclave, in fact, serves to heat the product contained in the load to a temperature greater than 100°C. In this way, within the bag containing the soaked cloths/mitts, steam is produced from the aqueous solution saturating the fabric. This internal steam denatures the proteins of any microorganisms present, killing them and providing the sterility of the product.

The modification of the values of temperature, pressure and exposure time, which currently are not included among those advised in the Pharmacopoeia, is therefore necessary in order to obtain the objective set by the present invention.

The data obtained from the present inventors confirm that it is possible to effectively sterilize the "wet" medical devices closed within plastic bags by means of the process described herein, also at a temperature of 105°C. This is very interesting from a commercial point of view:
- It makes it possible to introduce the wet steam sterilization process for numerous thermolabile devices.
- It makes it possible to reduce the use of the sterilization to ETO with clear benefits also from an environmental perspective.

Thus, the present invention relates to a process for the sterilization of cloths, gauze and/or mitts comprising the following steps:
- introducing in an autoclave one or more cloths, gauze and/or mitts soaked with an aqueous solution, wherein said cloths, gauze and/or mitts are closed in a container/packaging whereby during the sterilization the aqueous solution generates steam inside of said container;
- performing the sterilization in the autoclave at about 105°C at a pressure of about 2 bar.

Preferred features of the invention are the subject of the dependent claims. Some of the advantages of the present invention are summarized hereinafter.
- It prevents sterilized materials from undergoing any deterioration, in spite of a longer treatment time.
- It broadens the range of materials that can be sterilized by steam sterilization.
- Plastic or heat-sensitive materials can be sterilized, whereas this was not the case previously.

A further important advantage associated with the process described herein is that the pressure of two bar, in contrast to the standard methodology, makes it possible to counteract the swelling/deterioration/explosion of the sealed contents. Traditionally, in the case of rigid containers, the stopper was left open so as to allow overpressure created as a result of the evaporation of the liquid internally, caused by the heat, to escape and not deform the container or cause it to explode. In the case of traditional practice, however, it is possible, or at the very least not ruled out, that the steam originating from the autoclave may come into contact, during the cooling phase, with the solution inside the container, modifying the parameters of the solution in an uncontrolled manner. In the process according to the invention, instead, there is no contact between the saturating liquid and the steam generated by the autoclave. Compared to known sterilization processes in an autoclave, a further advantage is therefore the absence of a risk of dilution of the solution, and contact with a steam which may contain undesirable ions, such as metals absorbed by the apparatus during the production of the steam itself, is avoided.

Other advantages and features of the present invention will become clear from the following detailed description.

### Detailed description of the invention

The present invention relates to a process for the sterilization of cloths, gauze and/or mitts.

In particular, the invention relates to the sterilization of cloths, gauze and/or mitts soaked with an aqueous solution having detergent and/or antiseptic properties. The aqueous solution used to saturate the cloths, gauze and/or mitts prior to the sterilization may comprise water in an amount equal to or greater than 90% by weight and compounds and/or active ingredients having detergent and/or antiseptic activity. The solutions comprise an extract of Aloe barbadensis and/or Linoleamidopropyl pg-dimonium chloride phosphate (Phospholipid EFA CAS No. 83682-78-4) and/or Allantoin and/or poloxamer or any combination thereof. For example, the medical devices may be suitable for the treatment of pressure sores and/or venous and arterial ulcers.

In accordance with a preferred embodiment of said cloths, gauze and/or mitts, they are made of non-woven fabric. The weight, the thickness, the composition and the type of non-woven fabric have no effect on the sterilization process. For example, the fabric can be subjected to sterilization regardless of whether it is a spunlace or needle-punched non-woven fabric. In addition, the sterilization process described herein is effective on 100% viscose fabrics, on 100% synthetic fabrics, and on viscose fabrics in combination with synthetic fibers such as polyesters or polyethylenes. Another advantage of the present invention is the fact that the sterilization process is applicable to various forms of product: in particular containers, for example bags, containing a single saturated cloth, gauze and/or mitt and also containers, for example bags, containing more than one of said items can be sterilized effectively. In particular, by means of the sterilization process according to the present invention, it is possible to effectively sterilize containers that contain more than one cloth, gauze and/or mitt. In one embodiment of the invention said container may contain two, three, four, five, six, seven, or eight cloths, gauzes and/or mitts. In other words the present invention also relates to a process for the sterilization, according to any one of the embodiments described herein, of cloths, gauze and/or mitts soaked by an aqueous solution and closed in a container, wherein said container may contain up to eight cloths, gauzes and/or mitts.

A further advantage of the sterilization process according to the present invention is that it does not include any alteration either of the form or content of the sterilized cloths, gauze or mitts.

In particular, the mitts described herein are obtained by means of the following production process:
1. the machine feeds the reel of non-woven fabric;
2. the non-woven fabric is folded in half;
3. the mitt is welded on its two long sides by means of ultrasound technology;
4. the mitt pass beneath a blade which cuts it and gives it the correct shaping;
5. the mitt is saturated by the solution prepared beforehand; and
6. the mitt is folded so as to then be packaged, individually or in a larger quantity, in the laminate bag and lastly sterilized.

The saturated steam sterilization according to the present invention does not in any way alter the shape or content of the mitt.

The process according to the present invention provides a first step in which the soaked cloths, gauze and/or mitts are introduced into an autoclave for the successive step of sterilization.

The products are introduced into the closed autoclave in a container such that, during the sterilization, the aqueous solution, for example an aqueous solution as defined above, generates steam within the container. The container can be, for example, a packaging made of plastic or heat-sensitive material. According to one embodiment the containers of the products are packaged in a heat-sealed manner with facilitated opening in heat-resistant multilayer material, in particular in a multi-layer plastic film with an aluminum layer. According to alternative embodiments laminate bags with a mixed composition of polyester, aluminum and polypropylene can be used. According to another embodiment a composite laminate bag comprising cast polypropylene can be used.

Once introduced, the product to be sterilized in the autoclave will undergo a step of sterilization in the autoclave at about 105°C at a pressure of about 2 bar for at least 30 minutes.

The term "about 105°C" in the present description indicates a tolerance of ± 2°C, preferably ± 1°C, whereas the term "2 bar" indicates a tolerance of ± 2 bar, preferably ± 0.1 bar.

In accordance with a preferred embodiment the following parameters are used in the autoclave:

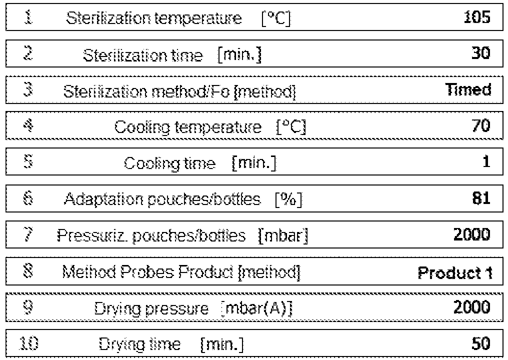

The parameters listed above are defined in Table 1 below:

**Table 1**

| **Parameter** | **Value** | **Description** |
|---|---|---|
| 1. Sterilization temperature [°C] | 105 | Temperature value maintained during the sterilization phase, measured by a probe positioned inside a product bag in the center of the load. |
| 2. Sterilization time [min.] | 30 | Duration in minutes of the sterilization phase. During this phase the temperature of the product is held at 105°C. |
| 3. Sterilization method | Timed | The setting specifies that the sterilization phase finishes when the time of the phase itself is ≥ the time set for parameter 2. |
| 4. Cooling temperature [°C] | 70 | The temperature that must be reached by the product at the end of the cooling phase. |
| 5. Cooling time [min.] | 1 | The waiting time, at the end of the cooling phase, after which the product has reached the temperature set for parameter 4. |
| 6. Adaptation pouches/bottles [%] | 81 | The parameter relates to the control of the overpressure of the chamber. The value set to 81 means that the pressure of the chamber adapts dynamically to the value of the temperature measured by the temperature probe of the product in order to bring the pressure to the value set for parameter 7. The regulation of the overpressure occurs in steam admission, sterilization and cooling phases. |
| 7. Pressuriz. Pouches/bottles [mbar] | 2000 | Value of overpressure set and maintained for the steam admission, sterilization and cooling phases. |
| 8. Method Probes Product [method] | Product 1 | The probe TE6 is active, that is to say the temperature probe that is positioned inside the load drives all the phases of the cycle. For example, only when the probe measures 105°C does the sterilization phase start. |
| 9. Drying pressure [mbar(A)] | 2000 | Value of overpressure maintained during the drying phase. |
| 10. Drying time [min] | 50 | Duration of the drying phase in minutes. |

### EXAMPLES

### EXAMPLE 1: Sterilization process

The sterilization procedure was performed on various medical products from WELCARE INDUSTRIES. These products were in the form of non-woven fabric CLOTHS or MITTS soaked with an aqueous solution having detergent or antiseptic properties, closed in heat-sealed packs with facilitated opening in multilayer material (plastic-aluminum film), suitable for and resistant to heat. The samples used to perform the tests were manufactured and packaged in accordance with the internal procedures of WELCARE INDUSTRIES and belonged to routine production batches. These products were, for example, Easyderm^{®} Sterile Glove code: 95950 and 95949, Easyderm^{®} Sterile Cloth code: 95600 and 95604 and UCS^{®} (code 95150). The process described hereinafter is particularly suitable for the sterilization of the product sold under the name UCS^{®} (code 95150), that is to say a single-use sterile gauze, soaked with a solution intended for the debridement of wounds, cleaning and hydrating the perilesional area and the edges of the wound, and cleaning of the entire affected limb. The product is provided packaged in a bag, sealed on four sides, with facilitated opening and made of a multilayer film with aluminum. The non-woven fabric cloth of the gauze, of the needle-punched kind, is formed of a combination of viscose, polyester and polypropylene, saturated with a solution based on poloxamer, Aloe Barbadensis leaf extract, and Allantoin.

The steam sterilization process for medical devices of the kind described herein from the manufacturer WELCARE INDUSTRIES was performed using an Air - Steam Mixture Autoclave, however the sterilization procedure is clearly applicable with any autoclave model validated in respect of its sterility by an authorized laboratory.

The sterilization parameters used and as defined beforehand in Table 1 are shown below:

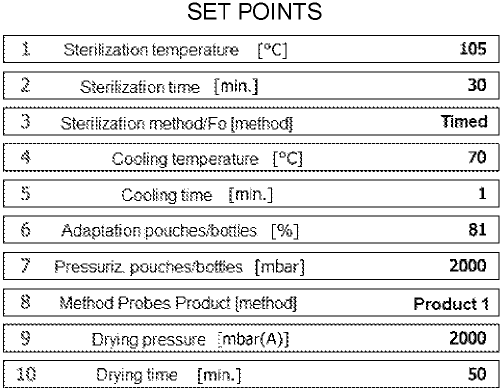

### EXAMPLE 2: Sterility test

The products sterilized in accordance with the procedure of Example 1 were subjected to sterility tests.

The sterility test was performed in accordance with the procedure described in standard UNI EN ISO 11737-2. In summary, the sample to be tested was immersed in a growth medium to promote the development of any bacteria that had resisted sterilization. At the end of the incubation period (about 2 weeks) it was checked whether or not microbial growth had occurred. If the test was positive (with growth), the sterilization process had been unsuccessful, and, if negative (no growth), the sterilization process had been successful. As defined by the reference standard, the absence of activity of the culture medium indicates the sterility of the analyzed sample. All the samples analyzed were sterile following the microbiological analyses. In spite of the lower temperatures during the tests, the Bowie-Dick tests (a technique used to check the correct functioning of an autoclave) always yielded positive results.

In particular, the ability to remove air and penetration of the steam in the load, which is correlated with its efficacy in the sterilization process, were verified. The test is based on the use of special card indicator which has the feature of transforming, that is to say changing color, when exposed to a certain pressure of saturated water vapor. The card is placed inside a closed container and is placed in the middle of the apparatus, and then the sterilization procedure is started. Correct functioning is confirmed if, at the end of the process, the card has transformed totally and uniformly. If not, this means that the sterilization of materials having cavities or empty hollows is not optimal. If a positive result is obtained for the Bowie-Dock test, this confirms that saturated steam is created at the used pressure and temperature values, this being an essential system property in order for sterilization to occur.

The data demonstrating the sterilization of the products from a certified company are shown below:
The results of the sterility tests performed on the products subjected to sterilization validation and of the biological indicators introduced within the validation cycles are shown below.

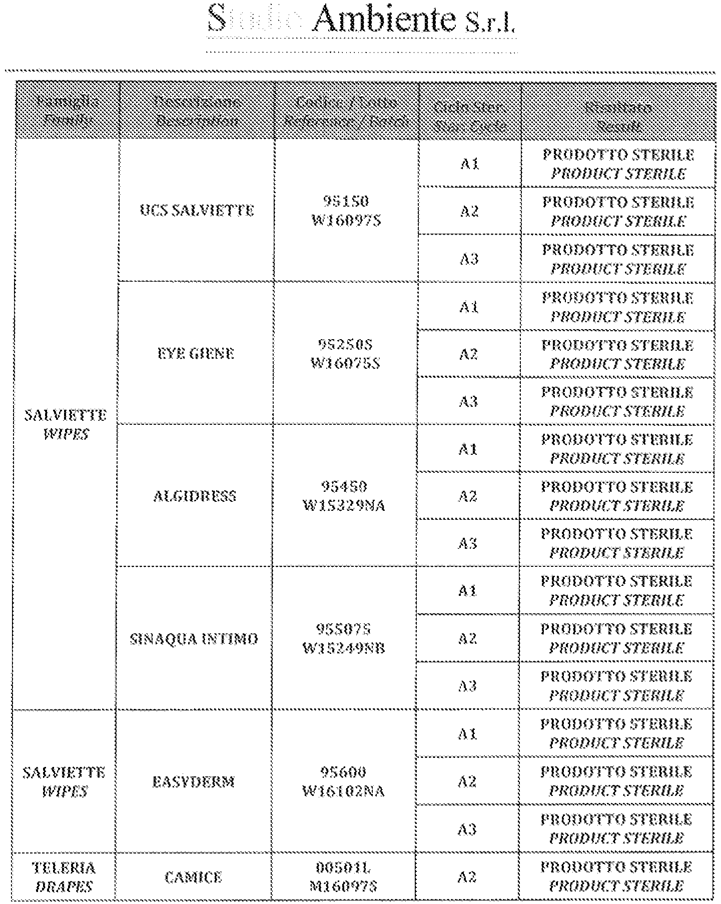

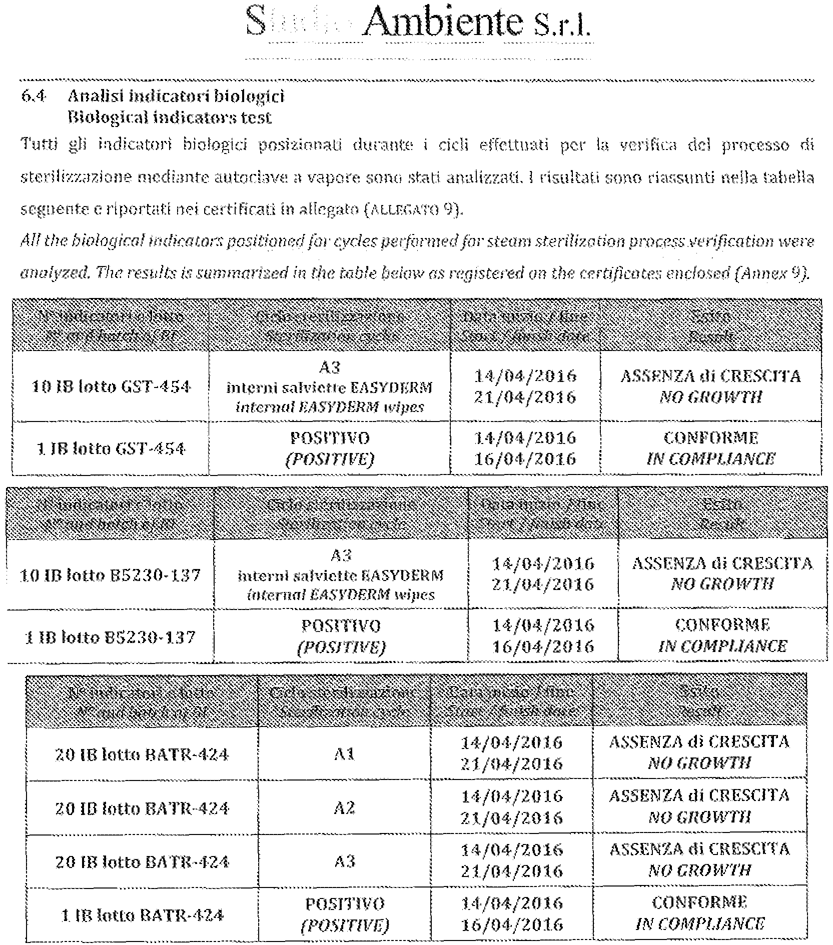

## Claims

1. A process for the sterilization of cloths, gauze and/or gloves without fingers for treatment of the skin comprising the following steps:
- introducing in an autoclave one or more cloths, gauze and/or gloves without fingers for treatment of the skin soaked of an aqueous solution, wherein said cloths, gauze and/or gloves without fingers for treatment of the skin are closed in a container whereby during the sterilization the aqueous solution generates steam inside of said container;
- perform the sterilization in autoclave at 105°C ± 2°C at a pressure of 2 bar ± 0.2 bar , wherein said aqueous solution has detergent and/or antiseptic properties and comprises an extract of Aloe barbadensis and/or Linoleamidopropyl pg-dimonium chloride phosphate (Phospholipid EFA CAS No. 83682- 78-4) and/or Allantoin and/or poloxamer.

2. The process according to claim 1, wherein the autoclave sterilization step is of at least 30 minutes.

3. The process according to any one of claims 1 to 2, wherein said container comprises up to eight cloths, gauze and/or gloves without fingers for treatment of the skin.

4. The process according to any one of claims 1 to 3, wherein said cloths, gauze and/or gloves without fingers for treatment of the skin are closed in containers made of plastic or materials that tend to deform and/or become damaged with rising temperature.

5. The process according to any one of claims 1 to 4, wherein said cloths, gauze and/or gloves without fingers for treatment of the skin are closed in containers which are heat-sealed with facilitated opening in multilayer material which is resistant to a temperature of 105°C ± 2°C without deformation or damage, in particular in a multi-layer plastic film with an aluminium layer.

6. The process according to any one of claims 1 to 5, wherein said cloths, gauze and/or gloves without fingers for treatment of the skin are closed in laminated bags with a mixed composition of polyester, aluminium and polypropylene.

7. The process according to any one of claims 1 to 6, wherein in the autoclave step the following parameters are used: 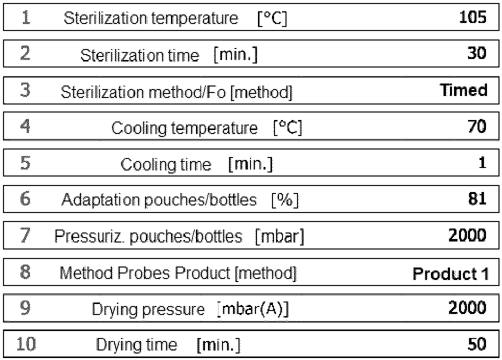 Wherein:
- Sterilization temperature is the temperature value maintained during the sterilization phase, measured by a probe positioned inside a product bag in the center of the load;
- Sterilization time is the duration in minutes of the sterilization phase. During this phase the temperature of the product is held at 105°C;
- Sterilization method is a setting that specifies that the sterilization phase finishes when the time of the phase itself is ≥ the time set for parameter 2.
- Cooling temperature is the temperature that must be reached by the product at the end of the cooling phase.
- Adaptation pouches/bottles is a parameter that relates to the control of the overpressure of the chamber. The value set to 81 means that the pressure of the chamber adapts dynamically to the value of the temperature measured by the temperature probe of the product in order to bring the pressure to the value set for parameter 7. The regulation of the overpressure occurs in steam admission, sterilization and cooling phases.
- Pressuriz. Pouches/bottles is the value of overpressure set and maintained for the steam admission, sterilization and cooling phases
- Method Probes Product is a parameters indicating that probe TE6 is active, that is to say the temperature probe that is positioned inside the load drives all the phases of the cycle.
- Drying pressure is the value of overpressure maintained during the drying phase.
- Dying time is the duration of the drying phase in minutes.

8. The process according to any one of claims 1 to 7, wherein said cloths, gauze and/or gloves without fingers for treatment of the skin are medical devices for the treatment of pressure sores and/or venous and arterial ulcers.

9. The process according to any one of claims 1 to 8, wherein said cloths, gauze and/or gloves without fingers for treatment of the skin are made of non-woven fabric.

## Patentansprüche

1. Prozess für die Sterilisation von Tüchern, Verbandsmullen und/oder fingerlosen Handschuhen für eine Behandlung der Haut, umfassend die folgenden Schritte:
- Einbringen eines oder mehrerer mit einer wässrigen Lösung getränkter Tücher, Verbandsmulle und/oder fingerloser Handschuhe für die Behandlung der Haut in einen Autoklaven, wobei die Tücher, Verbandsmulle und/oder fingerlosen Handschuhe für die Behandlung der Haut in einem Behälter verschlossen sind, wobei während der Sterilisation die wässrige Lösung im Inneren des Behälters Dampf erzeugt;
- Durchführen der Sterilisation in dem Autoklaven bei 105 °C ± 2 °C bei einem Druck von 2 bar ± 0,2 bar, wobei die wässrige Lösung reinigende und/oder antiseptische Eigenschaften aufweist und einen Extrakt aus Aloe barbadensis und/oder Linoleamidopropyl-pg-dimoniumchloridphosphat (Phospholipid EFA CAS-Nr. 83682-78-4) und/oder Allantoin und/oder Poloxamer umfasst.

2. Prozess nach Anspruch 1, wobei der Autoklavsterilisationsschritt mindestens 30 Minuten dauert.

3. Prozess nach einem der Ansprüche 1 bis 2, wobei der Behälter bis zu acht Tücher, Verbandsmulle und/oder fingerlose Handschuhe für die Behandlung der Haut umfasst.

4. Prozess nach einem der Ansprüche 1 bis 3, wobei die Tücher, Verbandsmulle und/oder fingerlosen Handschuhe für die Behandlung der Haut in Behältern verschlossen sind, die aus Kunststoff oder Materialien bestehen, die dazu neigen, sich bei steigender Temperatur zu verformen und/oder beschädigt zu werden.

5. Prozess nach einem der Ansprüche 1 bis 4, wobei die Tücher, Verbandsmulle und/oder fingerlosen Handschuhe für die Behandlung der Haut in Behältern verschlossen sind, die mit erleichterter Öffnung in einem mehrschichtigen Material heißversiegelt sind, das einer Temperatur von 105 °C ± 2 °C ohne Verformung oder Beschädigung standhält, insbesondere in einer mehrschichtigen Kunststofffolie mit einer Aluminiumschicht.

6. Prozess nach einem der Ansprüche 1 bis 5, wobei die Tücher, Verbandsmulle und/oder fingerlosen Handschuhe für die Behandlung der Haut in laminierten Beuteln aus einer Mischzusammensetzung aus Polyester, Aluminium und Polypropylen verschlossen werden.

7. Prozess nach einem der Ansprüche 1 bis 6, wobei in dem Autoklavenschritt die folgenden Parameter verwendet werden: 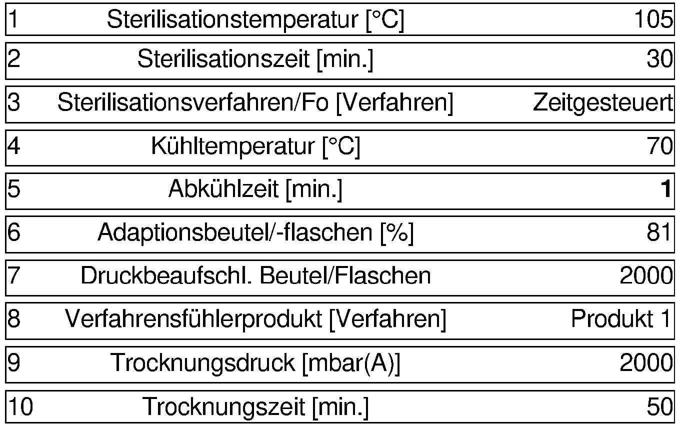 Wobei:
- Sterilisationstemperatur der Temperaturwert ist, der während der Sterilisationsphase aufrechterhalten wird, gemessen durch einen Fühler, der im Inneren eines Produktbeutels in der Mitte der Ladung positioniert ist;
- Sterilisationszeit die Dauer der Sterilisationsphase in Minuten ist. Während dieser Phase wird die Temperatur des Produkts bei 105 °C gehalten;
- Sterilisationsverfahren eine Einstellung ist, die spezifiziert, dass die Sterilisationsphase beendet ist, wenn die Zeit der Phase selbst ≥ die für Parameter 2 eingestellte Zeit ist.
- Kühltemperatur die Temperatur ist, die durch das Produkt am Ende der Kühlphase erreichen muss.
- Adaptionsbeutel/-flaschen ein Parameter ist, der sich auf die Kontrolle des Überdrucks der Kammer bezieht. Der auf 81 eingestellte Wert bedeutet, dass sich der Druck in der Kammer an den durch den Temperaturfühler des Produkts gemessenen Temperaturwert dynamisch anpasst, um den Druck auf den für Parameter 7 eingestellten Wert zu bringen. Die Regulierung des Überdrucks erfolgt in der Dampfzuführungs-, Sterilisations- und Kühlphase.
- druckbeaufschl. Beutel/Flaschen der für die Dampfzuführungs-, Sterilisations- und Kühlphase eingestellte und aufrechterhaltene Wert des Überdrucks ist.
- Verfahrensfühlerprodukt ein Parameter ist, der angibt, dass der Fühler TE6 aktiv ist, nämlich der Temperaturfühler, der sich im Inneren der Ladung befindet, alle Phasen des Zyklus steuert.
- Trocknungsdruck der Überdruckwert ist, der während der Trocknungsphase aufrechterhalten wird.
- Trocknungszeit die Dauer der Trocknungsphase in Minuten ist.

8. Prozess nach einem der Ansprüche 1 bis 7, wobei die Tücher, Verbandsmulle und/oder fingerlose Handschuhe für die Behandlung der Haut medizinische Vorrichtungen für die Behandlung von Druckgeschwüren und/oder venösen und arteriellen Geschwüren sind.

9. Prozess nach einem der Ansprüche 1 bis 8, wobei die Tücher, Verbandsmulle und/oder fingerlosen Handschuhe für die Behandlung der Haut aus Vliesstoff bestehen.

## Revendications

1. Procédé de stérilisation de linges, gazes et/ou gants sans doigts pour le traitement de la peau, comprenant les étapes suivantes :
- l'introduction dans un autoclave un ou plusieurs linges, gazes et/ou gants sans doigts pour le traitement de la peau imbibés d'une solution aqueuse, dans lequel lesdits linges, gazes et/ou gants sans doigts pour le traitement de la peau sont fermés dans un conteneur, selon lequel la solution aqueuse génère de la vapeur à l'intérieur dudit conteneur pendant la stérilisation ;
- le fait d'effectuer la stérilisation en autoclave à 105 °C ± 2 °C à une pression de 2 bar ± 0,2 bar, dans lequel ladite solution aqueuse a des propriétés détergentes et/ou antiseptiques et comprend un extrait d'Aloe barbadensis et/ou du phosphate de chlorure de linoléamidopropyl pg-dimonium (Phospholipide EFA CAS No. 83682- 78-4) et/ou de l'Allantoïne et/ou du poloxamère.

2. Procédé selon la revendication 1, dans lequel l'étape de stérilisation en autoclave dure au moins 30 minutes.

3. Procédé selon l'une quelconque des revendications 1 à 2, dans lequel ledit récipient comprend jusqu'à huit linges, gazes et/ou gants sans doigts pour le traitement de la peau.

4. Procédé selon l'une quelconque des revendications 1 à 3, dans lequel lesdits linges, gazes et/ou gants sans doigts pour le traitement de la peau sont enfermés dans des récipients en plastique ou dans des matériaux qui ont tendance à se déformer et/ou à s'abîmer avec l'augmentation de la température.

5. Procédé selon l'une quelconque des revendications 1 à 4, dans lequel lesdits linges, gazes et/ou gants sans doigts pour le traitement de la peau sont enfermés dans des récipients thermoscellés avec ouverture facilitée dans un matériau multicouche résistant à une température de 105 °C ± 2 °C sans déformation ni dommage, en particulier dans un film plastique multicouche avec une couche d'aluminium.

6. Procédé selon l'une quelconque des revendications 1 à 5, dans lequel lesdits linges, gazes et/ou gants sans doigts pour le traitement de la peau sont fermés dans des sacs laminés avec une composition mixte de polyester, d'aluminium et de polypropylène.

7. Procédé selon l'une quelconque des revendications 1 à 6, dans lequel les paramètres suivants sont utilisés dans l'étape de l'autoclave : 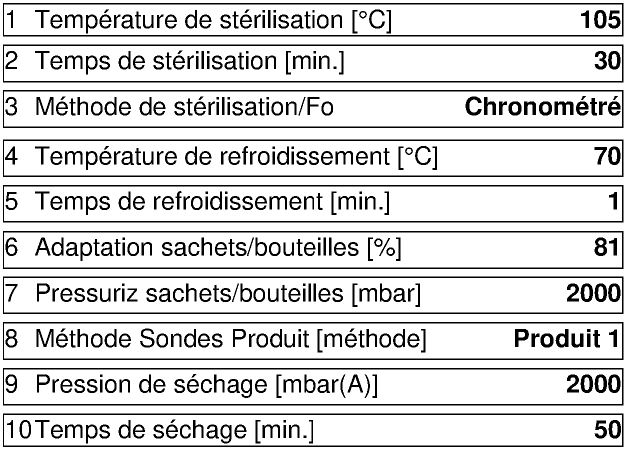 dans lequel :
- La température de stérilisation est la valeur de la température maintenue pendant la phase de stérilisation, mesurée par une sonde placée à l'intérieur d'un sac de produit au centre de la charge ;
- Le temps de stérilisation est la durée en minutes de la phase de stérilisation. Pendant cette phase, la température du produit est maintenue à 105 °C ;
- La méthode de stérilisation est un réglage qui spécifie que la phase de stérilisation se termine lorsque le temps de la phase elle-même est ≥ le temps défini pour le paramètre 2.
- La température de refroidissement est la température que doit atteindre le produit à la fin de la phase de refroidissement.
- Les sachets/bouteilles d'adaptation sont un paramètre lié au commande de la surpression de la chambre. La valeur fixée à 81 signifie que la pression de la chambre s'adapte dynamiquement à la valeur de la température mesurée par la sonde de température du produit afin d'amener la pression à la valeur fixée pour le paramètre 7. La régulation de la surpression s'effectue lors des phases d'admission de la vapeur, de stérilisation et de refroidissement.
- Pressuriz. Sachets/bouteilles est la valeur de la surpression fixée et maintenue pour les phases d'admission de la vapeur, de stérilisation et de refroidissement
- Méthode Sondes Le produit est un paramètre indiquant que la sonde TE6 est active, c'est-à-dire que la sonde de température positionnée à l'intérieur de la charge pilote toutes les phases du cycle.
- La pression de séchage est la valeur de la surpression maintenue pendant la phase de séchage.
- Le temps de séchage est la durée de la phase de séchage en minutes.

8. Procédé selon l'une quelconque des revendications 1 à 7, dans lequel lesdits linges, gazes et/ou gants sans doigts pour le traitement de la peau sont des dispositifs médicaux pour le traitement des escarres et/ou des ulcères veineux et artériels.

9. Procédé selon l'une quelconque des revendications 1 à 8, dans lequel les linges, gazes et/ou les gants sans doigts pour le traitement de la peau sont en tissu non tissé.
